# EUROPÄISCHE PATENTSCHRIFT

(11) **EP 1 960 328 B1**
(45) Veröffentlichungstag und Bekanntmachung des Hinweises auf die Patenterteilung: **06.04.2011**
(21) Anmeldenummer: 06829076.6
(22) Anmeldetag: 18.11.2006
(51) Int. Cl.: C07C 2/86

(54) **VERFAHREN ZUR SUBSTITUTION VON INDENOFLUORENEN**
METHOD FOR SUBSTITUTING INDENOFLUORENES
PROCEDE POUR SUBSTITUER DES INDENOFLUORENES

(30) Priorität: 17.12.2005 DE 102005060438
(43) Veröffentlichungstag der Anmeldung: 27.08.2008
(73) Patentinhaber: Merck Patent GmbH, 64293 Darmstadt (DE)
(72) Erfinder: BUESING, Arne, 65959 Frankfurt/Main (DE)
(86) Internationale Anmeldenummer: PCT/EP2006/011086
(87) Internationale Veröffentlichungsnummer: WO 2007/068326

(56) Entgegenhaltungen:
- EP-A- 1 491 568
- R ANÉMIAN ET AL: "Monodisperse fluorene oligomers exhibiting strong dipolar coupling interactions" CHEMICAL COMMUNICATIONS., 2002, Seiten 1608-1609, XP002433068 GBCHEMICAL SOCIETY, LONDON.
- J PEI ET AL: "Star-shaped Polycyclic aromatics based on oligothiophene-functionalized truxene: synthesis, properties and facile emissive wavelength tuning & SUPPORTING INFORMATION" JOURNAL OF THE AMERICAN CHEMICAL SOCIETY., Bd. 125, Nr. 33, 2003, Seiten 9944-S13, XP002433069 XXXX

## Beschreibung

Die vorliegende Erfindung betrifft ein neues Verfahren zur Herstellung von substituierten Indenofluorenen, die insbesondere Verwendung in organischen, elektronischen Vorrichtungen finden.

Der Einsatz organischer, halbleitender Verbindungen, die zur Emission von Licht im sichtbaren Spektralbereich befähigt sind, in organischen Elektrolumineszenzvorrichtungen (OLEDs) steht gerade am Anfang der Markteinführung. Der allgemeine Aufbau derartiger Vorrichtungen wird beispielsweise in der US 4.539.507, der US 5.151.629, der EP 0676461 und der WO 98/27136 beschrieben.

Als organische, halbleitende Verbindungen, die zur Emission von Licht im sichtbaren Spektralbereich befähigt sind, werden u. a. auch organische Verbindungen und Polymere mit Indenofluoren-Substrukturen verwendet. Zur Verbesserung der anwendungstechnischen Eigenschaften kann es von Vorteil sein, den Indenofluoren-Grundkörper, beispielsweise durch Einführung geeigneter Substituenten, zu modifizieren.

Aus dem Stand der Technik sind Verfahren zur Substitution von Fluoren-Grundkörpern bekannt (Kelley et al., J. Chem. Res. Miniprint 1997, 12, 2701-2733; Anemian et al., Chem. Commun. 2002, 15, 1608-1609), bei denen eine Alkylierung an einem Fluoren-Grundkörper in Gegenwart von Kalium-tert-butylat in Dimethylformamid (DMF) durchgeführt wird. Wird dieses aus dem Stand der Technik bekannte Verfahren auf Indenofluoren-Grundkörper übertragen, werden Ausbeuten von bis zu 84 % und Reinheiten von bis zu 93 % erhalten.

Des Weiteren ist aus dem Stand der Technik ein zweistufiges Verfahren zur Substitution von Indenofluoren-Grundkörpern bekannt (Macromolecules 2000, 33, 2016-2020), bei dem eine Alkylierung an einem Indenofluoren-Grundkörper in Gegenwart von n-Butyl-Lithium in THF bei tiefen Temperaturen durchgeführt wird. Bei diesem Verfahren werden Reinheiten von bis zu 99 % erreicht; die Ausbeute des zweistufigen Verfahren beträgt bis zu 84 %. Aufgrund der Reaktionsbedingungen und der gewählten Reagenzien ist die Herstellung der substituierten Indenofluoren-Grundkörper relativ teuer. Weiterhin stellen Verfahren mit Alkyllithium-Reagenzien wegen der hohen Entzündlichkeit ein Sicherheitsrisiko im technischen Maßstab dar.

Aus der EP 1491568 A1 ist ein Verfahren zur Alkylierung von cis-Indenofluoren bekannt, bei dem die Alkylierung des Indenofluorens in Gegenwart von Butyllithium in THF bei tiefen Temperaturen durchgeführt wird.

J. Pei et al. (J. Am. Chem. Soc. 2003, 125, 9944-9945) beschreiben ein Verfahren zur Alkylierung von Truxen, bei dem die Alkylierung des Truxens in Gegenwart von n-Butyllithium in THF bei tiefen Temperaturen durchgeführt wird.

Es besteht also weiterhin der Bedarf für ein Verfahren zur Herstellung substituierter Indenofluoren-Grundkörper, das einerseits die Zielverbindungen in hoher Ausbeute und hoher Reinheit zur Verfügung stellt und andererseits den Zugang in einer kostengünstigen und sicherheitstechnisch unkritischen Weise erlaubt.

Es wurde nunmehr überraschend gefunden, dass substituierte Indenofluoren-Grundkörper in hoher Ausbeute und Reinheit zugänglich sind, wenn die Substitution in Gegenwart ausgewählter Basen und Lösungsmittel durchgeführt wird.

Gegenstand der Erfindung ist somit ein Verfahren zur Herstellung von trans-Indenofluorenverbindungen der Formel (Ia) bzw. cis-Indenofluoren-Verbindungen der Formel (Ib), 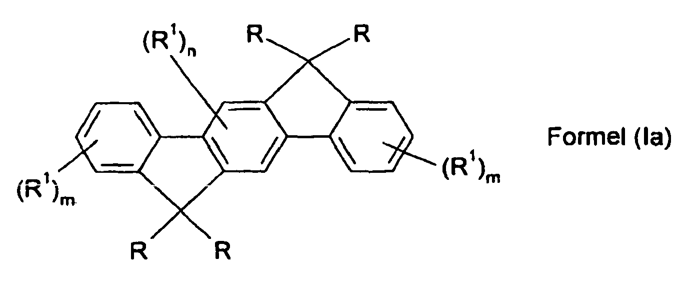 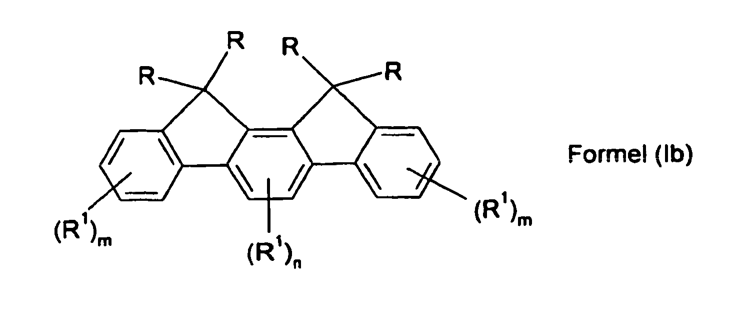 durch Umsetzung mindestens einer Verbindung der Formel (IIa) bzw. (IIb) 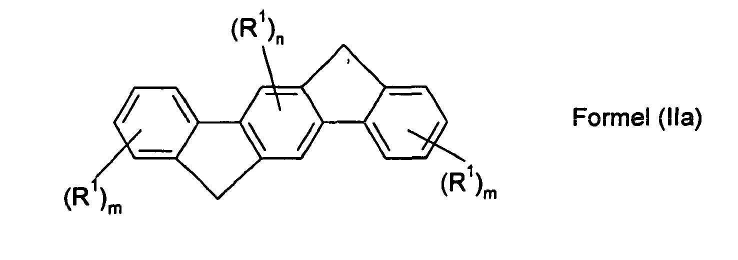 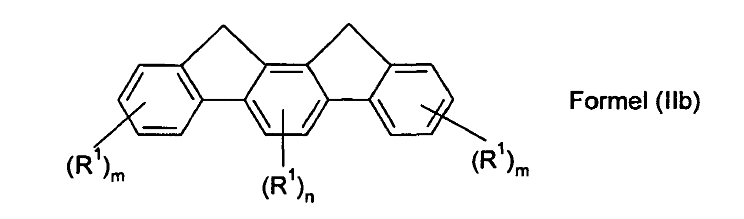 mit mindestens einer Verbindung der Formel (III)

R-Hal Formel (III)

in Gegenwart mindestens einer organischen Base, wobei als organische Base ein Metallalkoholat eingesetzt wird, und mindestens eines organischen, polaren, aprotischen Lösungsmittels.

Der Index n steht unabhängig voneinander für eine ganze Zahl 0, 1 oder 2.

Der Index m steht unabhängig voneinander für eine ganze Zahl 0, 1, 2, 3 oder 4.

Bei den Verbindungen der Formel (la), (Ib), (IIa) und (IIb) können ein oder mehrere aromatische Kohlenstoffatome durch Heteroatome, insbesondere durch N, ersetzt sein.

Des Weiteren können die Verbindungen der Formel (la), (Ib), (IIa) und (IIb) am aromatischen Grundkörper weitere, unter den Reaktionsbedingungen inerte, Substituenten R¹ tragen.

Bei diesen inerten Substituenten R¹, die bei jedem Auftreten gleich oder verschieden sind, handelt es sich um Wasserstoff, Fluor, Chlor, Brom, Iod, eine geradkettige Alkyl-, Alkoxy- oder Thioalkoxygruppe mit 1 bis 40 C-Atomen, eine verzweigte Alkyl-, Alkoxy- oder Thioalkoxygruppe mit 3 bis 40 C-Atomen, eine cyclische Alkyl-, Alkoxy- oder Thioalkoxygruppe mit 3 bis 40 C-Atomen oder eine Alkenylgruppe mit 2 bis 40 C-Atomen, wobei die vorstehend genannten Alkyl-, Alkoxy-, Thioalkoxy- oder Alkenyl-Reste mit einem oder mehreren Resten R² substituiert sein können und wobei eine oder mehrere nicht benachbarte CH₂-Gruppen durch -R²C=CR²-, -C≡C-, Si(R²)₂, Ge(R²)₂, Sn(R²)₂, NR², C=O, P(=O)R², C=S, C=Se, C=NR². -O-, -S- oder -CONR²- ersetzt sein können und ein oder mehrere H-Atome durch F ersetzt sein können, oder ein aromatisches oder heteroaromatisches Ringsystem mit 5 bis 40 aromatischen Ringatomen, das durch einen oder mehrere Reste R² substituiert sein kann, oder eine Aryloxy- oder Heteroaryloxygruppe mit 5 bis 40 aromatischen Ringatomen, die durch einen oder mehrere Reste R² substituiert sein kann, oder eine Diarylaminogruppe mit 12 bis 40 aromatischen Ringatomen, die durch einen oder mehrere Reste R² substituiert sein kann, oder eine Kombination dieser Systeme; dabei können zwei oder mehrere Substituenten R¹ auch miteinander ein mono- oder polycyclisches, aliphatisches oder aromatisches, Ringsystem, beispielsweise ein kondensiertes Benzosystem, bilden.

Der Rest R² ist bei jedem Auftreten gleich oder verschieden H oder ein aliphatischer oder aromatischer Kohlenwasserstoffrest mit 1 bis 20 C-Atomen.

Der Rest Hal steht für eine Abgangsgruppe, insbesondere eine organische Abgangsgruppe und/oder ein Halogenatom. Als organische Abgangsgruppen werden vorzugsweise Tosylat, Mesylat, Trifluormethansulfonat, Pyridinium, Tetraalkylammonium, Benzolsulfonat und/oder Nitrophenolat verstanden, als Halogene werden vorzugsweise Cl, Br und/oder I verstanden.

Der Rest R steht für eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R² substituiert sein kann, wobei jeweils eine oder mehrere nicht benachbarte CH₂-Gruppen durch -R²C=CR²-, -C≡C-, Si(R²)₂, Ge(R²)₂, Sn(R²)₂, C=O, C=S, C=Se, C=NR², -O-, -S- oder -CONR²-ersetzt sein können und wobei ein oder mehrere H-Atome durch F ersetzt sein können, oder ein aromatisches oder heteroaromatisches Ringsystem mit 5 bis 40 aromatischen Ringatomen, das durch einen oder mehrere Reste R² substituiert sein kann, oder eine Aryloxy- oder Heteroaryloxygruppe mit 5 bis 40 aromatischen Ringatomen, die durch einen oder mehrere Reste R² substituiert sein kann, oder eine Kombination dieser Systeme. Bevorzugt steht der Rest R für eine geradkettige Alkylgruppe mit 1 bis 20 C-Atomen oder für eine verzweigte oder cyclische Alkylgruppe mit 3 bis 20 C-Atomen, die jeweils mit einem oder mehreren Resten R² substituiert sein kann, wobei jeweils eine oder mehrere nicht benachbarte CH₂-Gruppen durch -R²C=CR²-, -C≡C-, Si(R²)₂, C=O, C=NR², -O-, -S- oder -CONR²- ersetzt sein können und wobei ein oder mehrere H-Atome durch F ersetzt sein können. Besonders bevorzugt steht R für eine geradkettige, verzweigte oder cyclische Alkylgruppe mit bis zu 10 C-Atomen.

Bevorzugt wird für die Reste R und R¹ unter einer aromatischen Gruppe bzw. heteroaromatischen Gruppe ein System mit einem gemeinsamen aromatischen Elektronensystem verstanden, wobei eine Arylgruppe 6 bis 24 C-Atome und eine Heteroarylgruppe 2 bis 24 C-Atome und insgesamt mindestens 5 aromatische Ringatome umfasst. Die Heteroatome sind vorzugsweise ausgewählt aus N, O und/oder S. Dies kann im Sinne dieser Erfindung ein einfacher Homo- oder Heterocyclus sein, beispielsweise Benzol, Pyridin, Thiophen, etc., oder es kann ein kondensiertes, aromatisches Ringsystem sein, in dem mindestens zwei aromatische oder heteroaromatische Ringe, beispielsweise Benzolringe, miteinander "verschmolzen". d. h. durch Anellierung einander ankondensiert sind, also mindestens eine gemeinsame Kante und dadurch auch ein gemeinsames aromatisches System aufweisen. Diese Aryl- oder Heteroarylgruppen können substituiert oder unsubstituiert sein; ebenso können gegebenenfalls vorhandene Substituenten weitere Ringsysteme bilden. So sind beispielsweise Systeme wie Naphthalin, Anthracen, Phenanthren, Pyren, etc. als Arylgruppen und Chinolin, Acridin, Benzothiophen, Carbazol, etc. als Heteroarylgruppen im Sinne dieser Erfindung zu sehen, während beispielsweise Biphenyl, Fluoren, Spirobifluoren, etc. keine Arylgruppen im Sinne dieser Erfindung darstellen, da es sich hierbei um separate, aromatische Elektronensysteme handelt.

Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nichtaromatische Einheit (weniger als 10 % der von H verschiedenen Atome, vorzugsweise weniger als 5 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, 9,9-Diarylfluoren, Triarylamin, Diarylether, etc. als aromatische Ringsysteme im Sinne dieser Erfindung verstanden werden.

Im Rahmen der vorliegenden Erfindung werden unter einer geradkettigen C₁- bis C₄₀-Alkylgruppe, einer verzweigten Alkylgruppe mit 3 bis 40 C-Atomen bzw. einer cyclischen Alkylgruppe mit 3 bis 40 C-Atomen solche Systeme verstanden, bei denen auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl und Octinyl verstanden.

Unter einer geradkettigen C₁- bis C₄₀-Alkoxygruppe, einer verzweigten Alkoxygruppe mit 3 bis 40 C-Atomen bzw. einer cyclischen Alkoxygruppe mit 3 bis 40 C-Atomen werden besonders bevorzugt Methoxy, Ethoxy, n-Propoxy, i-Propoxy, n-Butoxy, i-Butoxy, s-Butoxy, t-Butoxy oder 2-Methylbutoxy verstanden.

Unter einer Aryl- oder Heteroarylgruppe, die je nach Verwendung monovalent oder bivalent sein kann, die noch jeweils substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol. Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol. Unter aromatischen und heteroaromatischen Ringsystemen im Sinne dieser Erfindung werden außer den oben genannten Aryl- und Heteroarylgruppen insbesondere Biphenylen, Terphenylen, Fluoren, Spirobifluoren, Dihydrophenanthren, Tetrahydropyren und cis- oder trans-Indenofluoren verstanden.

Bevorzugt sind Verbindungen, in denen das Symbol R¹ gleich oder verschieden bei jedem Auftreten für H steht und/oder in denen mindestens ein Symbol R¹ gleich oder verschieden bei jedem Auftreten für eine geradkettige Alkylgruppe mit 1 bis 10 C-Atomen, vorzugsweise 1 bis 8 C-Atomen, oder eine verzweigte Alkylgruppe mit 3 bis 10 C-Atomen, vorzugsweise 3 bis 8 C-Atomen, wobei eine oder mehrere nicht benachbarte CH₂-Gruppen durch -R²C=CR²-, -C≡C-, -O- oder -S- ersetzt sein können und wobei ein oder mehrere H-Atome durch F ersetzt sein können, oder eine monovalente Aryl- oder Heteroarylgruppe mit 5 bis 16 aromatischen Ringatomen, die mit einem oder mehreren Resten R² substituiert sein kann, steht, besonders bevorzugt für Methyl, tert-Butyl oder eine monovalente Aryl- oder Heteroarylgruppe mit 4 bis 6 C-Atomen, die mit einem oder mehreren Resten R² substituiert sein kann, oder eine Diarylaminogruppe mit 12 bis 20 C-Atomen, die mit einem oder mehreren Resten R² substituiert sein kann.

Bei den organischen Basen handelt es sich insbesondere um Natrium- und Kalium-Alkoholate. Bevorzugt sind Natrium- und Kalium-Alkoholate mit 1 bis 8 Kohlenstoffatomen, insbesondere tertiäre Natrium- und/oder Kalium-Alkoholate. Besonders bevorzugt sind Natrium-tert-butanolat und Kalium-tert-butanolat. Die Aufzählung geeigneter organischer Basen ist an dieser Stelle nicht abschließend, vielmehr umfasst der Begriff organische Basen" auch andere an dieser Stelle nicht genannte Verbindungen, welche für das erfindungsgemäße Verfahren gleichermaßen geeignet sind.

Als organische, polare, aprotische Lösungsmittel werden vorzugsweise Dimethylsulfoxid (DMSO), N-Methylpyrrolidon (NMP), Dimethylformamid (DMF), Dimethylacetamid, Acetonitril, Aceton oder Butanon eingesetzt, bevorzugt DMSO oder DMF, besonders bevorzugt DMSO.

Im Rahmen des erfindungsgemäßen Verfahrens liegt das Mischungsverhältnis von Verbindungen der Formel (III) zu organischer Base vorzugsweise zwischen 1:10 und 1:1, und besonders bevorzugt zwischen 1:6 und 1:3.

Die Anfangskonzentration von Verbindungen der Formel (IIa) bzw. (IIb) im Lösemittel beträgt vorzugsweise maximal 1 M, besonders bevorzugt maximal 0,5 M, und insbesondere maximal 0,25 M.

Das erfindungsgemäße Verfahren wird vorzugsweise in einem Temperaturbereich von 0 °C bis 100 °C betrieben. Zur Vervollständigung der Reaktion kann die Reaktionsmischung auf Temperaturen von bis zu 150 °C erwärmt werden.

Im Rahmen des erfindungsgemäßen Verfahrens wird die Base vorzugsweise zusammen mit der Ausgangsverbindung der Formel (IIa) bzw. (IIb) vorgelegt und die Reaktionstemperatur durch die Geschwindigkeit der Zugabe der Verbindung der Formel (III) gesteuert. Die Zugabe der Verbindungen der Formel (III) erfolgt vorzugsweise bei Temperaturen zwischen 0 °C und 100 °C.

Das erfindungsgemäße Verfahren wird vorzugsweise unter Inertgas, beispielsweise Stickstoff oder einem Edelgas, wie z.B. Neon oder Argon, betrieben. Vorzugsweise wird das Verfahren unter Normaldruck betrieben. Es kann jedoch auch unter erhöhtem oder reduziertem Druck durchgeführt werden.

Die Reaktion ist in der Regel, je nach Absatzgröße, zwischen 15 und 30 Minuten nach Zugabe des Reagenzes R-Hal abgeschlossen, wobei jedoch in dieser Angabe keine Beschränkung des Verfahrens gesehen werden soll.

Im Rahmen des erfindungsgemäßen Verfahrens kann es von Vorteil sein, die Reaktion durch dem Fachmann an sich bekannte Hilfsmittel zu beschleunigen oder gar in Gang zu bringen. Als geeignete Hilfsmittel sind dem Fachmann der Einsatz von Ultraschall, Mikrowellen und/oder anderen Aktivierungen, beispielsweise Zugabe von Iodid oder Silbersalzen bekannt.

Die im Rahmen der Erfindung eingesetzten polaren, aprotischen, organischen Lösungsmittel sollten hinreichend getrocknet und ggf. auch entgast sein. Vorzugsweise liegt der Restwassergehalt bei maximal 0,05 Gew.-% Wasser (bestimmt nach Karl Fischer).

Mit Hilfe des erfindungsgemäßen Verfahrens sind die Zielverbindungen der Formel (Ia) bzw. (Ib) in guter Ausbeute und hoher Reinheit auch in kommerziell benötigten Mengen in wirtschaftlicher Weise herstellbar. Weiterhin stellen die verwendeten Reagenzien sicherheitstechnisch kein Risiko dar.

Die Ausbeuten des erfindungsgemäßen Verfahrens betragen vorzugsweise mindestens 90 % bezogen auf die eingesetzte(n) Ausgangsverbindung(en) der Formel (IIa) bzw. (IIb).

Die mittels des erfindungsgemäßen Verfahrens hergestellten Verbindungen können beispielsweise als Monomere bzw. Comonomere zur Erzeugung entsprechender konjugierter, teilkonjugierter oder nichtkonjugierter Polymere, Oligomere oder auch als Kern von Dendrimeren Verwendung finden. Die Polymerisation erfolgt dabei in der Regel über eine noch einzuführende Halogenfunktionalität.

Weiterhin können die alkylierten Verbindungen, nach Einführung einer Halogenfunktionalität, durch eine Hartwig-Buchwald-Kupplung in aromatische Amine überfährt werden, die beispielsweise als Lochtransportmaterialien Anwendung finden können, oder durch eine Suzuki-Kupplung mit weiteren aromatischen Gruppen gekuppelt werden. Diese Verbindungen können beispielsweise als Host für organische Dotanden Anwendung finden.

Die Erfindung wird im Folgenden anhand von Ausführungsbeispielen näher erläutert, ohne dadurch jedoch eingeschränkt zu werden.

### Beispiele

### Beispiel 1: Verstellung von Tetraoctyl-trans-indenofluoren

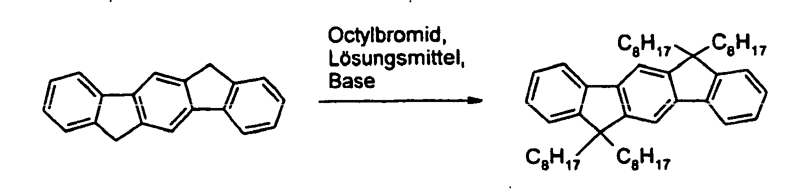

38,7 g (152 mmol) Indenofluoren werden in einem ausgeheizten Kolben in 600 ml getrocknetem DMSO gelöst. Bei Raumtemperatur werden 87,8 g (914 mmol) NaOtBu zugegeben. Die nunmehr blaue Suspension wird auf eine Innentemperatur von 80 °C gebracht. Bei dieser Temperatur werden zur jetzt violetten Lösung 158 ml (914 mmol) 1-Octylbromid so zugetropft, dass die Innentemperatur 90 °C nicht übersteigt (Dauer: ca. 30 Minuten). Der Ansatz wird weitere 30 Minuten bei 80 bis 90 °C Innentemperatur gehalten, dann auf 1500 ml Eiswasser gegossen und ca. 20 Minuten gerührt. Der ausgefallene Feststoff wird abgesaugt und nacheinander mit ca. 200 ml H₂O und Methanol gewaschen. Die Ausbeute beträgt 104,9 g (98 %) bei einer HPLC-Reinheit von 99,5 %.

### Beispiel 2: Herstellung von Tetramethyl-trans-indenofluoren

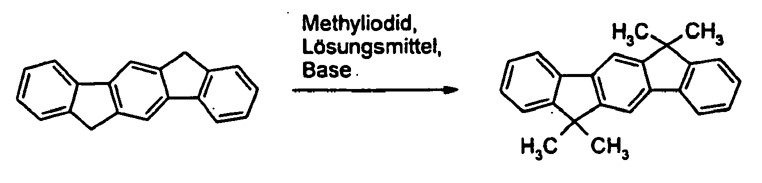

39.0 g (153 mmol) Indenofluoren werden in einem ausgeheizten Kolben in 1900 mL getrocknetem DMSO gelöst. Bei Raumtemperatur werden 117.6 g (1224 mmol) NaOtBu zugegeben. Die Suspension wird auf eine Innentemperatur von 65 °C gebracht. Nach 30 min werden 76.2 ml (1224 mmol) Mel in 100 mL trockenem DMSO zugetropft und 7 h bei dieser Temperatur gerührt. Nach Zugabe von 100 mL konz. NH₄OH-Lösung wird das Gemisch mit 1500 mL Wasser versetzt, der Feststoff abgesaugt, mit insgesamt 2000 mL Wasser gewaschen und im Vakuum getrocknet. Die Ausbeute beträgt 45.5 g (96%) bei einer Reinheit von 99.8%.

### Beispiele 3 bis 5:

### Herstellung von Tetraoctyl-trans-indenofluoren

Die Herstellung erfolgt analog zu Beispiel 1, wobei die in Tabelle 1 aufgeführten Reaktionsbedingungen eingehalten wurden und die in der Tabelle aufgeführten Ergebnisse erhalten wurden.

**Tabelle 1**

| Beispiel | Bedingungen (8 Äquiv. Octylbromid) | Ausbeute | Reinheit Rohprodukt |
|---|---|---|---|
| 3 | KOtBu, DMF, 4 h, 90 °C | 84 % | 93 % |
| 4 | KOtBu, DMSO, 4 h, 90 °C | 98 % | 97 % |
| 5 | NaOtBu, DMF, 4 h, 90 °C | 84 % | 94 % |

### Vergleichsbeispiele 6 bis 8:

### Herstellung von Tetraoctyl-trans-indenofluoren

Die Herstellung erfolgt analog zu Beispiel 1, wobei die in Tabelle 2 aufgeführten Reaktionsbedingungen eingehalten wurden und die in der Tabelle aufgeführten Ergebnisse erhalten wurden.

**Tabelle 2**

| Vergleichsbeispiel | Bedingungen (8 Äquiv. Octylbromid) | Ausbeute | Reinheit Rohprodukt |
|---|---|---|---|
| 6 | NaOH 50 %,Bu₄NBr, 4 d, 90 °C | 25 % | 80 % |
| 7 | NaH, DMSO, 4 d, 90 °C | 60 % | 85 % |
| 8 | n-BuLi,¹⁾ THF, -75 °C → RT | 84 % | 97 % |

| | | | |
|---|---|---|---|
| ¹⁾ Durchführung in zwei Zyklen | | | |

Wie man aus den erfindungsgemäßen und den Vergleichsbeispielen erkennen kann, erhält man mit dem erfindungsgemäßen Verfahren bessere Ausbeuten und höhere Reinheiten des Rohprodukts als mit Verfahren gemäß dem Stand der Technik. Dies gilt in ganz besonderem Maße, wenn DMSO als Lösemittel verwendet wird.

## Patentansprüche

1. Verfahren zur Herstellung von trans-Indenofluorenverbindungen der Formel (Ia) bzw. cis-Indenofluoren-Verbindungen der Formel (Ib), 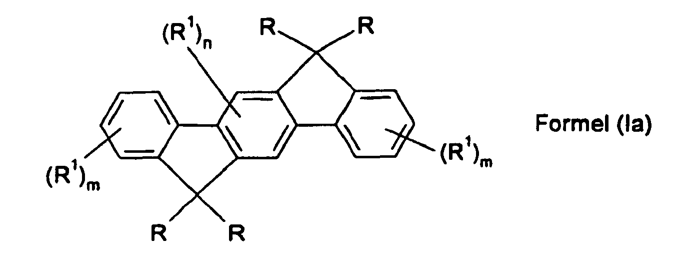 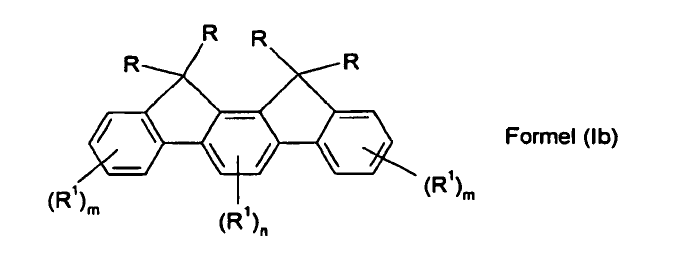 durch Umsetzung mindestens einer Verbindung der Formel (IIa) bzw. (IIb) 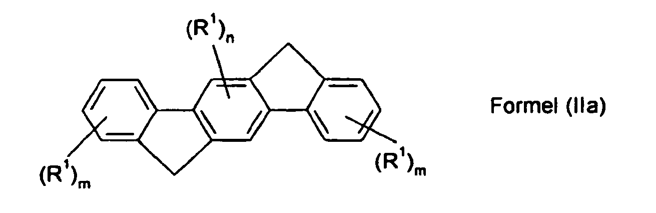 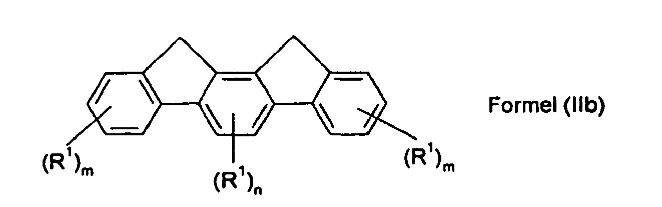 mit mindestens einer Verbindung der Formel (III)
R-Hal Formel (III)
in Gegenwart mindestens einer organischen Base, wobei als organische Base ein Metall-Alkoholat eingesetzt wird, und mindestens eines organischen, polaren, aprotischen Lösungsmittels,
wobei die Reste R, R¹ und Hal folgende Bedeutung haben:
R¹ ist bei jedem Auftreten gleich oder verschieden, und steht für Wasserstoff, Fluor, Chlor, Brom, Iod, eine geradkettige Alkyl-, Alkoxy- oder Thioalkoxygruppe mit 1 bis 40 C-Atomen, eine verzweigte Alkyl-, Alkoxy- oder Thioalkoxygruppe mit 3 bis 40 C-Atomen, eine cyclische Alkyl-, Alkoxy- oder Thioalkoxygruppe mit 3 bis 40 C-Atomen oder eine Alkenylgruppe mit 2 bis 40 C- Atomen, wobei die vorstehend genannten Alkyl-, Alkoxy-, Thioalkoxy- oder Alkenyl-Reste mit einem oder mehreren Resten R² substituiert sein können und wobei eine oder mehrere nicht benachbarte CH₂-Gruppen durch -R²C=CR²-, -C≡C-, Si(R²)₂, Ge(R²₎₂, Sn(R²)₂, NR², C=O, P(=O)R², C=S, C=Se, C=NR², -O-, -S- oder -CONR²- ersetzt sein können und wobei ein oder mehrere H-Atome durch F ersetzt sein können, oder ein aromatisches oder heteroaromatisches Ringsystem mit 5 bis 40 aromatischen Ringatomen, das durch einen oder mehrere Reste R² substituiert sein kann, oder eine Aryloxy- oder Heteroaryloxygruppe mit 5 bis 40 aromatischen Ringatomen, die durch einen oder mehrere Reste R² substituiert sein kann, oder eine Diarylaminogruppe mit 12 bis 40 aromatischen Ringatomen, die durch einen oder mehrere Reste R² substituiert sein kann, oder eine Kombination dieser Systeme; dabei können zwei oder mehrere Substituenten R¹ auch miteinander ein mono- oder polycyclisches aliphatisches oder aromatisches Ringsystem bilden,
Hal steht für eine Abgangsgruppe, insbesondere eine organische Abgangsgruppe und-/oder ein Halogenatom,
R ist bei jedem Auftreten gleich oder verschieden, und steht für eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch -R²C=CR²-, -C≡C-, Si(R²)₂, Ge(R²)₂, Sn(R²)_{2,} NR², C=O, C=S, C=Se, C=NR², -O-, -S- oder -CONR²- ersetzt sein können und wobei ein oder mehrere H-Atome durch F ersetzt sein können, oder ein aromatisches oder heteroaromatisches Ringsystem mit 5 bis 40 aromatischen Ringatomen, das durch einen oder mehrere Reste R² substituiert sein kann, oder eine Aryloxy- oder Heteroaryloxy-gruppe mit 5 bis 40 aromatischen Ringatomen, die durch einen oder mehrere Reste R² substituiert sein kann, oder eine Kombination dieser Systeme,
R² ist bei jedem Auftreten gleich oder verschieden H oder ein aliphatischer oder aromatischer Kohlenwasserstoffrest mit 1 bis 20 C-Atomen,
n steht unabhängig voneinander für eine ganze Zahl 0, 1 oder 2, und
m steht unabhängig voneinander für eine ganze Zahl 0, 1, 2, 3 oder 4.

2. Verfahren nach Anspruch 1, **dadurch gekennzeichnet, dass** ein oder mehrere aromatische Kohlenstoffatome in den Verbindungen der Formel (la), (IIb), (IIa) und (IIb) durch Heteroatome, insbesondere durch N, ersetzt sind.

3. Verfahren nach Anspruch 1 oder 2, **dadurch gekennzeichnet, dass** R¹ unter den Reaktionsbedingungen inert ist.

4. Verfahren nach einem oder mehreren der Ansprüche 1 bis 3, **dadurch gekennzeichnet, dass** das Symbol R gleich oder verschieden bei jedem Auftreten für eine geradkettige Alkylgruppe mit 1 bis 20 C-Atomen oder für eine verzweigte oder cyclische Alkylgruppe mit 3 bis 20 C-Atomen, die jeweils mit einem oder mehreren Resten R² substituiert sein kann, wobei jeweils eine oder mehrere nicht benachbarte CH₂-Gruppen durch -R²C=CR²-, -C≡C-, Si(R²)₂, C=O, C=NR², -O-, -S- oder -CONR²- ersetzt sein können und wobei ein oder mehrere H-Atome durch F ersetzt sein können, steht.

5. Verfahren nach einem oder mehreren der Ansprüche 1 bis 4, **dadurch gekennzeichnet, dass** als organische Basen Natrium- und Kalium-Alkoholate eingesetzt werden.

6. Verfahren nach einem oder mehreren der Ansprüche 1 bis 5, **dadurch gekennzeichnet, dass** das Mischungsverhältnis von Verbindungen der Formel (III) zu organischer Base zwischen 1:10 und 1:1 liegt.

7. Verfahren nach einem oder mehreren der Ansprüche 1 bis 6, **dadurch gekennzeichnet, dass** die Anfangskonzentration von Verbindungen der Formel (IIa) bzw. (IIb) im Lösungsmittel maximal 1 M beträgt.

8. Verfahren nach einem oder mehreren der Ansprüche 1 bis 7, **dadurch gekennzeichnet, dass** das Verfahren in einem Temperaturbereich von 0 °C bis 100 °C betrieben wird.

9. Verfahren nach einem oder mehreren der Ansprüche 1 bis 8, **dadurch gekennzeichnet, dass** die Zugabe der Verbindung der Formel (III) bei Temperaturen zwischen 0 °C und 100 °C erfolgt.

10. Verfahren nach einem oder mehreren der Ansprüche 1 bis 9, **dadurch gekennzeichnet, dass** als Lösungsmittel Dimethylsulfoxid, N-Methylpyrrolidon, Dimethylformamid, Dimethylacetamid, Acetonitril, Aceton oder Butanon eingesetzt wird, insbesondere Dimethylsulfoxid.

11. Verfahren nach einem oder mehreren der Ansprüche 1 bis 10, **dadurch gekennzeichnet, dass** das Lösungsmittel einen Restwassergehalt von maximal 0,05 Gew.-% Wasser (bestimmt nach Karl Fischer) aufweist.

## Claims

1. Process for the preparation of trans-indenofluorene compounds of the formula (Ia) or cis-indenofluorene compounds of the formula (Ib), 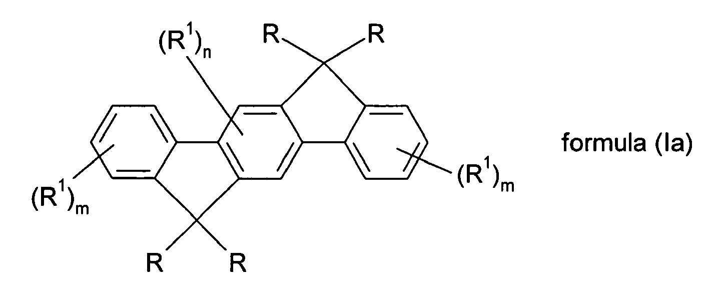 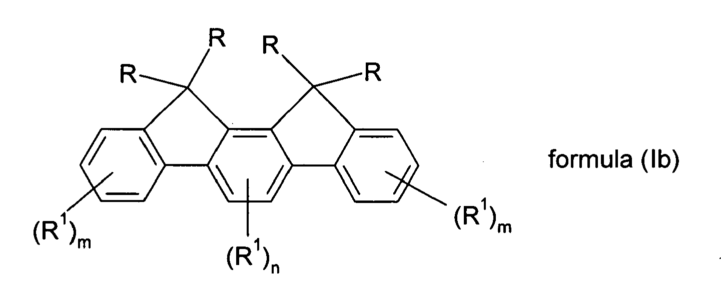 by reaction of at least one compound of the formula (IIa) or (IIb) respectively, 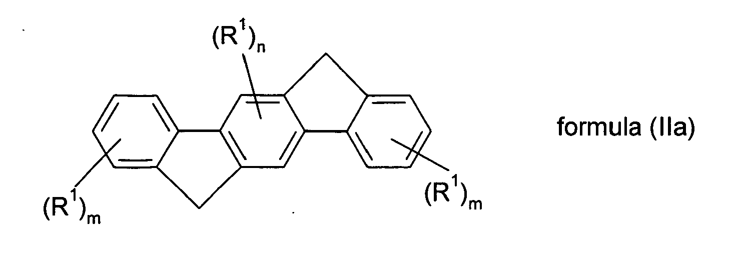 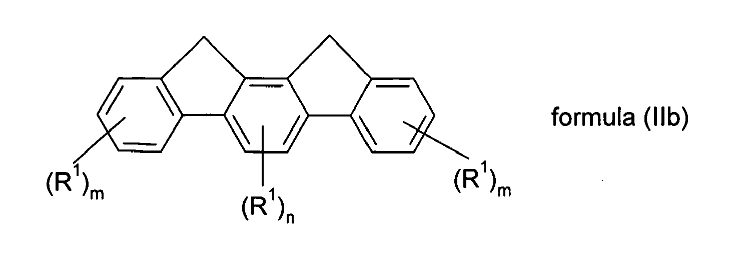 with at least one compound of the formula (III),
R-Hal formula (III)
in the presence of at least one organic base, where the organic base employed is a metal alkoxide, and at least one organic, polar, aprotic solvent,
where the radicals R, R¹ and Hal have the following meaning:
R¹ is identical or different on each occurrence and stands for hydrogen, fluorine, chlorine, bromine, iodine, a straight-chain alkyl, alkoxy or thioalkoxy group having 1 to 40 C atoms, a branched alkyl, alkoxy or thioalkoxy group having 3 to 40 C atoms, a cyclic alkyl, alkoxy or thioalkoxy group having 3 to 40 C atoms or an alkenyl group having 2 to 40 C atoms, where the above-mentioned alkyl, alkoxy, thioalkoxy or alkenyl radicals may be substituted by one or more radicals R² and where one or more non-adjacent CH₂ groups may be replaced by -R²C=CR²-, -C≡C-, Si(R²)₂, Ge(R²)₂, Sn(R²)₂, NR^{2,} C=O, P(=O)R², C=S_{,} C=Se, C=NR², -O-, -S- or -CONR²- and where one or more H atoms may be replaced by F, or an aromatic or heteroaromatic ring system having 5 to 40 aro- matic ring atoms, which may be substituted by one or more radicals R², or an aryloxy or heteroaryloxy group having 5 to 40 aromatic ring atoms, which may be substituted by one or more radicals R², or a diarylamino group having 12 to 40 aromatic ring atoms, which may be substituted by one or more radicals R² or a combination of these systems; two or more substituents R¹ here may also form a mono- or poly- cyclic, aliphatic or aromatic ring system,
Hal stands for a leaving group, in particular an organic leaving group and/or a halogen atom,
R is identical or different on each occurrence and stands for a straight-chain alkyl, alkoxy or thioalkoxy group having 1 to 40 C atoms or a branched or cyclic alkyl, alkoxy or thioalkoxy group having 3 to 40 C atoms, each of which may be substi- tuted by one or more radicals R², where one or more non- adjacent CH₂ groups may be replaced by -R²C=CR² -, -C≡C-, Si(R²)₂, Ge(R²)₂, Sn(R²)₂, NR², C=O, C=S, C=Se, C=NR², -O-, -S- or -CONR²- and where one or more H atoms may be replaced by F, or an aromatic or heteroaromatic ring system having 5 to 40 aromatic ring atoms, which may be substituted by one or more radicals R², or an aryloxy or heteroaryloxy group having 5 to 40 aromatic ring atoms, which may be sub- stituted by one or more radicals R², or a combination of these systems,
R² is on each occurrence, identically or differently, H or an ali- phatic or aromatic hydrocarbon radical having 1 to 20 C atoms,
n stands, independently of one another, for an integer 0, 1 or 2, and
m stands, independently of one another, for an integer 0, 1, 2, 3 or 4.

2. Process according to Claim 1, **characterised in that** one or more aromatic carbon atoms in the compounds of the formulae (Ia), (Ib), (IIa) and (IIb) are replaced by heteroatoms, in particular by N.

3. Process according to Claim 1 or 2, **characterised in that** R¹ is inert under the reaction conditions.

4. Process according to one or more of Claims 1 to 3, **characterised in that** the symbol R, identically or differently on each occurrence, stands for a straight-chain alkyl group having 1 to 20 C atoms or for a branched or cyclic alkyl group having 3 to 20 C atoms, each of which may be substituted by one or more radicals R² where one or more non-adjacent CH₂ groups may each be replaced by -R²C=CR²-, -C=C-, Si(R²)₂, C=O, C=NR², -O-, -S- or -CONR²- and where one or more H atoms may be replaced by F.

5. Process according to one or more of Claims 1 to 4, **characterised in that** the organic bases employed are sodium alkoxides and potassium alkoxides.

6. Process according to one or more of Claims 1 to 5, **characterised in that** the mixing ratio of compounds of the formula (III) to organic base is between 1:10 and 1:1.

7. Process according to one or more of Claims 1 to 6, **characterised in that** the initial concentration of compounds of the formula (IIa) or (IIb) in the solvent is at most 1 M.

8. Process according to one or more of Claims 1 to 7, **characterised in that** the process is operated at a temperature in the range from 0°C to 100°C.

9. Process according to one or more of Claims 1 to 8, **characterised in that** the compound of the formula (III) is added at temperatures between 0°C and 100°C.

10. Process according to one or more of Claims 1 to 9, **characterised in that** the solvent employed is dimethyl sulfoxide, N-methylpyrrolidone, dimethylformamide, dimethylacetamide, acetonitrile, acetone or butanone, in particular dimethyl sulfoxide.

11. Process according to one or more of Claims 1 to 10, **characterised in that** the solvent has a residual water content of at most 0.05% by weight of water (determined by the Karl Fischer method).

## Revendications

1. Procédé de préparation de composés de trans-indénofluorène de formule (la) ou de composés de cis-indénofluorène de formule (Ib), 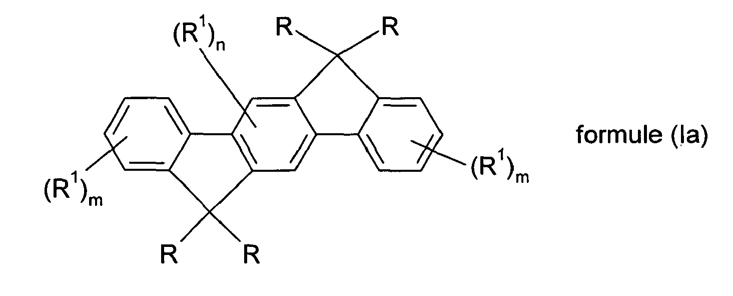 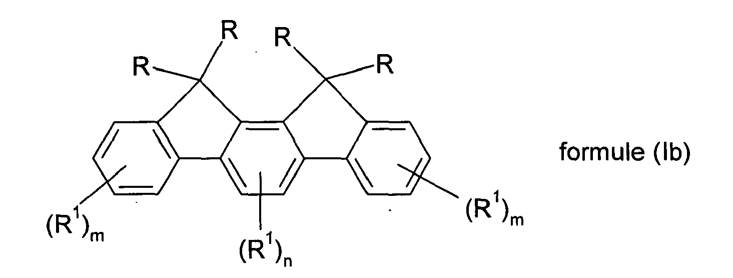 par réaction d'au moins un composé de formule (IIa) ou (IIb) respectivement, 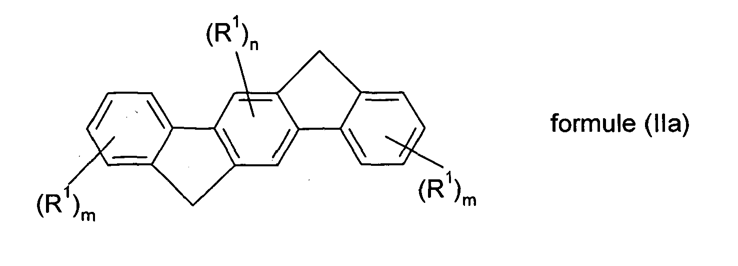 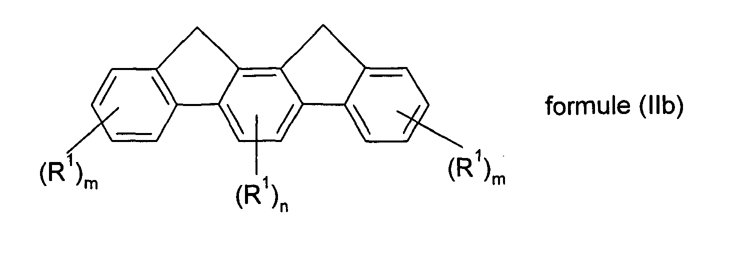 avec au moins un composé de formule (III),
R-Hal formule (III)
en présence d'au moins une base organique, où la base organique employée est un alcoxyde de métal, et au moins un solvant aprotique polaire organique,
où les radicaux R, R¹ et Hal revêtent la signification suivante :
R¹ est identique ou différent à chaque occurrence et représente hydrogène, fluor, chlore, brome, iode, un groupement alkyle, alcoxy ou thioalcoxy à chaîne linéaire ayant 1 à 40 atomes de C, un groupement alkyle, alcoxy ou thioalcoxy ramifié ayant 3 à 40 atomes de C, un groupement alkyle, alcoxy ou thio- alcoxy cyclique ayant 3 à 40 atomes de C ou un groupement alcényle ayant 2 à 40 atomes de C, où les radicaux alkyle, alcoxy, thioalcoxy ou alcényle susmentionnés peuvent être substitués par un ou plusieurs radicaux R² et où un ou plu- sieurs groupements CH₂ non adjacents peuvent être rempla- cés par -R²C=CR²-, -C≡C-, Si(R²)2, Ge(R²)2, Sn(R²)₂, NR², C=O, P(=O)R², C=S, C=Se, C=NR², -O-, -S- ou -CONR²- et où un ou plusieurs atomes de H peuvent être remplacés par F, ou un noyau aromatique ou hétéroaromatique ayant 5 à 40 atomes de cycle aromatique, qui peut être substitué par un ou plusieurs radicaux R², ou un groupement aryloxy ou hété- roaryloxy ayant 5 à 40 atomes de cycle aromatique, qui peut être substitué par un ou plusieurs radicaux R², ou un grou- pement diarylamino ayant 12 à 40 atomes de cycle aroma- tique, qui peut être substitué par un ou plusieurs radicaux R², ou une combinaison de ces systèmes ; deux, ou plus, subs- tituants R¹ peuvent ici aussi former un noyau mono- ou poly- cyclique, aliphatique ou aromatique,
Hal représente un groupement partant, en particulier un groupe- ment partant organique et/ou un atome d'halogène,
R est identique ou différent à chaque occurrence et représente un groupement alkyle, alcoxy ou thioalcoxy à chaîne linéaire ayant 1 à 40 atomes de C ou un groupement alkyle, alcoxy ou thioalcoxy ramifié ou cyclique ayant 3 à 40 atomes de C, chacun d'entre eux pouvant être substitué par un ou plu- sieurs radicaux R², où un ou plusieurs groupements CH₂ non adjacents peuvent être remplacés par -R²C=CR²-, -C≡C-, Si(R²)₂, Ge(R²)₂, Sn(R²)₂, NR², C=O, C=S, C=Se, C=NR², -O-, -S- ou -CONR²- et où un ou plusieurs atomes de H peu- vent être remplacés par F, ou un noyau aromatique ou hété- roaromatique ayant 5 à 40 atomes de cycle aromatique, qui peut être substitué par un ou plusieurs radicaux R², ou un groupement aryloxy ou hétéroaryloxy ayant 5 à 40 atomes de cycle aromatique, qui peut être substitué par un ou plusieurs radicaux R², ou une combinaison de ces systèmes,
R² est à chaque occurrence, de manière identique ou différente, H ou un radical hydrocarboné aliphatique ou aromatique ayant 1 à 20 atomes de C,
n représente, indépendamment l'un de l'autre, un nombre entier valant 0, 1 ou 2, et
m représente, indépendamment l'un de l'autre, un nombre entier valant 0, 1, 2, 3 ou 4.

2. Procédé selon la revendication 1, **caractérisé en ce qu'**un ou plusieurs atomes de carbone aromatiques dans les composés de formules (la), (Ib), (IIa) et (IIb) sont remplacés par des hétéroatomes, en particulier par N.

3. Procédé selon la revendication 1 ou 2, **caractérisé en ce que** R¹ est inerte dans les conditions réactionnelles.

4. Procédé selon l'une ou plusieurs des revendications 1 à 3, **caractérisé en ce que** le symbole R, de manière identique ou différente à chaque occurrence, représente un groupement alkyle à chaîne linéaire ayant 1 à 20 atomes de C ou un groupement alkyle ramifié ou cyclique ayant 3 à 20 atomes de C, chacun d'entre eux pouvant être substitué par un ou plusieurs radicaux R², où un ou plusieurs groupements CH₂ non adjacents peuvent chacun être remplacés par -R²C=CR²-, -C≡C-, Si(R²)₂, C=O, C=NR², -O-, -S- ou -CONR²- et où un ou plusieurs atomes de H peuvent être remplacés par F.

5. Procédé selon l'une ou plusieurs des revendications 1 à 4, **caractérisé en ce que** les bases organiques employées sont les alcoxydes de sodium et les alcoxydes de potassium.

6. Procédé selon l'une ou plusieurs des revendications 1 à 5, **caractérisé en ce que** le rapport de mélange des composés de formule (III) par rapport à la base organique est compris entre 1:10 et 1:1.

7. Procédé selon l'une ou plusieurs des revendications 1 à 6, **caractérisé en ce que** la concentration initiale en composés de formule (IIa) ou (IIb) dans le solvant est d'au plus 1 M.

8. Procédé selon l'une ou plusieurs des revendications 1 à 7, **caractérisé en ce que** le procédé est mis en oeuvre à une température dans la plage allant de 0°C à 100°C.

9. Procédé selon l'une ou plusieurs des revendications 1 à 8, **caractérisé en ce que** le composé de formule (III) est ajouté à des températures comprises entre 0°C et 100°C.

10. Procédé selon l'une ou plusieurs des revendications 1 à 9, **caractérisé en ce que** le solvant employé est le diméthylsulfoxyde, la N-méthylpyrrolidone, le diméthylformamide, le diméthylacétamide, l'acétonitrile, l'acétone ou la butanone, en particulier le diméthylsulfoxyde.

11. Procédé selon l'une ou plusieurs des revendications 1 à 10, **caractérisé en ce que** le solvant possède une teneur en eau résiduelle d'au plus 0,05% en poids d'eau (déterminé par la méthode de Karl Fischer).
